Europäisches Patentamt

(19) European Patent Office

Office européen des brevets

(11) EP 0 460 149 B1

(12) **EUROPEAN PATENT SPECIFICATION**

(45) Date of publication and mention
of the grant of the patent:
**10.12.1997 Bulletin 1997/50**

(21) Application number: **91900559.5**

(22) Date of filing: **25.10.1990**

(51) Int. Cl.$^6$: **A61K 7/025**, A61K 7/021,
A61K 6/00

(86) International application number:
**PCT/US90/06148**

(87) International publication number:
**WO 91/06277 (16.05.1991 Gazette 1991/11)**

(54) **PIGMENTED COSMETIC COMPOSITIONS AND METHOD OF MAKING SAME**

PIGMENTIERTE KOSMETISCHE ZUSAMMENSETZUNG UND HERSTELLUNGSVERFAHREN

COMPOSITIONS COSMETIQUES PIGMENTEES ET PROCEDE DE PRODUCTION

(84) Designated Contracting States:
**AT BE CH DE DK ES FR GB GR IT LI LU NL SE**

(30) Priority: **25.10.1989 US 426204**
**30.11.1989 US 443262**

(43) Date of publication of application:
**11.12.1991 Bulletin 1991/50**

(73) Proprietor: **AVON PRODUCTS, INC.**
**New York, NY 10019 (US)**

(72) Inventors:
• PAHLCK, Harold, E.
Waldwick, NJ 07463 (US)
• MARTIN, Shari, R.
Suffern, NY 10901 (US)
• SQUIRES, Michael, E.
Mahwah, NJ 07430 (US)

(74) Representative:
Müller, Hans-Jürgen, Dipl.-Ing. et al
Müller, Schupfner & Gauger
Postfach 10 11 61
80085 München (DE)

(56) References cited:
LU-A- 57 903          US-A- 4 390 524
US-A- 4 756 906

• PATENT ABSTRACTS OF JAPAN, vol. 12, no. 479 (C-552), 14th December 1988; & JP- A-63 196 505 (SHISEIDO CO., LTD) 15-08-1988

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

TECHNICAL FIELD

The present invention relates generally to cosmetic preparations and more specifically, to novel pigmented compositions having "activatable" dormant colored particles or pigments dispersed throughout a cosmetic vehicle or base. The cosmetic vehicle or base may comprise lipstick, blush, face powder, foundation, eyeshadow or any other cosmetic preparation containing a colorant or pigment. In use, shear forces (e.g. rubbing onto the surface of the skin) activate the dormant color component causing a sustained release of pigment thereby giving the original color a renewed intensity or a "long wearing" affect. Alternatively, sustained release of the dormant pigment may lend an unusual but aesthetic look to the cosmetic base composition characterized by a juxtaposition of at least two or more different colors or by the creation of a new color comprising a blend of the original color and the released pigment.

BACKGROUND ART

U.S. Patent No. 3,947,571 relates to the use of a microencapsulated oil in lipstick. Moisture applied to the lips after the lipstick has been applied results in a sustained release of oil from the capsules thus imparting a shiny "wet" look to the lips. This patent does not suggest microencapsulating pigment per se, nor does it suggest activating an encapsulated or dormant pigment by effecting rupture of the microcapsules via applied shear forces in order to achieve a "long wearing" affect.

U.S. Patent No. 4,752,496 relates to the microencapsulation of "never dry" or paste cosmetics using the well known conservation technique. After encapsulation the "greasy" cosmetic is applied to a substrate and subsequently overlaid with a film forming agent. The substrate may be used as samples capable of being distributed to consumers through the mail, or on inserts in magazines. Although this patent does suggest microencapsulating a "lipstick" formulation, it does not disclose nor suggest microencapsulation of a pigment in a cosmetic formulation (e.g. lipstick) for the purpose of rendering it dormant so that it subsequently may be activated to achieve a "long wearing" affect.

An Information Bulletin published by Dow Corning (Form No. 24-550-88) entitled "Information About specialty Copolymers" describes a highly cross-linked polymethacrylate copolymer in the form of a fine particle-sized, freely flowing powder which may be used to absorb various liquid systems both volatile and non-volatile used in cosmetic formulations. The Information Bulletin states that the Dow Corning polymer, sold under the designation POLYTRAP Q5-6603, may be spread into a thin film on a surface, such as rubbing on the skin, to cause the absorbed fluids to come into contact with such surface. The Dow Corning Information Bulletin however, fails to suggest loading the polymethacrylate copolymer with a pigment so that the latter may be rendered dormant until "activated" or released on a sustained basis for renewing the color in a cosmetic formulation and achieving a "long wearing" affect.

U.S. Patent No. 4,756,906 relates to a cosmetic colorant composition which relies on the use of microcapsules to add a second colorant to a first and different colorant. The intended effect of the added microcapsules is to render colored, highlight, or alter in color an existing color. That is, the intent is to effect a visible change in color. This intent is significantly different from providing a color identical or complementary to an original color to achieve a „long wearing" effect without any visible color change to the user.

PAOJ vol.12/no.479 (C-552) 14th December 1988 discloses a make-up cosmetic capable of controlling applied color by degree of force in coating the skin with the cosmetic or by number of times of embrocations, by blending a cosmetic with a non-rigid resin capsule collapsing by compression, containing a colored component and an oil component as inner contents. Here, the latter may be "activated" by mechanical forces so as to release its contents and sustain the applied color or alter the color. Nevertheless, there are no features or measures described which are capable to maintain a "long wearing" affect over a long period of time without any decrease in color contrast or „shiny" characteristics.

Past attempts to provide acceptable cosmetic products having pigmented solid particles which may be activated or released on a sustained basis by the application of shear forces thereby giving a renewed color intensity or "long wearing" look have not been successful. When pigments were microencapsulated and placed directly into a cosmetic base, it was found that the microcapsules felt "gritty and hard" on the skin, thus rendering the composition unacceptable as a cosmetic product. Attempts to ameliorate this undesirable condition by mixing the solid particles with oils or emollients of various types to form a dispersion also resulted in failure by yielding microcapsules that were fragile, and thus too easily rupturable during handling, or pigment concentrations insufficient to give the desired renewed color intensity. When pigment was added to fine particle-size agglomerated powders combined with a cosmetic vehicle or base and then rubbed into the skin, intensification of color was inadequate to provide a "long wearing" affect.

These disadvantages were overcome by the present invention when it was discovered that in a first form cosmetically acceptable compositions may be produced having microencapsulated pigmented solid particles by forming a dispersion of the pigment in a hydrophobic, non-volatile, low viscosity liquid carrier; coacervating the dispersion to yield microcapsules in the form of a stable, freely flowing, dry powder; and then incorporating the microcapsules in a compatible anhydrous base or vehicle. In a second or alternatively preferred form of the invention, it was discovered that the

2

pigment could be loaded onto a fine particle-size, high surface area, solid substrate and then coated with an adhering oil or outer film to entrap the pigment on the substrate. When the resulting cosmetic compositions were applied to the skin and rubbed or otherwise subjected to shear forces, the microcapsules and/or entrapping substrate particles were readily ruptured or otherwise mechanically distorted thereby releasing their intensely colored pigment and giving the compositions' color or shade a perceptible "long wearing" look.

The invention is disclosed in claim 1 and the subclaims refer to preferred embodiments.

The present invention overcomes the disadvantages of prior art and sustains a perceptible "long wearing" look by a medium which is additionally dispersed in a base or vehicle wherein the medium is capable of entrapping and releasing, or vice versa, pigments or dyes by means of a solid substrate like solid particulates. Therefore, the color pigments are rendered activatable after the microcapsules were subjected to physical forces since the medium entrappes superfluous pigments and ensures a unique and smooth perceptible "long wearing" look by releasing these pigments, if necessary, later on.

## DISCLOSURE OF THE INVENTION

Briefly described, the present invention contemplates cosmetic formulations having dispersed therein rupturable microcapsules the cores of which comprise pigmented solid particles dispersed in a hydrophobic, non-volatile, low-viscosity liquid carrier. The compositions are made by first forming the initial dispersion by mixing the pigmented solid particles with the liquid carrier, grinding the mixture to yield a uniform particle size distribution in the initial dispersion, and then microencapsulating the pigment/liquid carrier dispersion by coacervation to yield microcapsules in the form of a stable, free flowing, dry powder. The microcapsules then are further processed by being dispersed in a compatible cosmetic vehicle or base.

In an alternative embodiment, the pigment is applied to an entrapping substrate in the form of fine solid particles having a high surface area. The pigmented substrate is then coated with a film thereby yielding a stable, free flowing, dry powder which may be further processed by being dispersed in a compatible cosmetics vehicle or base.

## BEST MODE FOR CARRYING OUT THE INVENTION

The present invention relates to cosmetic formulations useful for imparting color to the surface of the skin. Such formulations, generally referred to as "makeup", may have many different forms including without limitation lipstick, foundation, face powder, blush, eyeshadow and so on. Each makeup form, in turn, generally includes a color component in the form of pigmented solid particles for giving it its characteristic color or "shade", or other solid particles for giving it a desired texture or sheen (mica, pearlescents, waxes, etc.) with the color component being dispersed throughout a suitable anhydrous base or vehicle. For example, in the case of "lipstick" the coloring agent or pigmented ingredients may be dispersed in a base comprising a mixture of waxes, emollients, and moisturizers, whereas, in the case of "blush", the pigmented solids may be dispersed in a base comprising a mixture of talc, kaolin, and various known binders.

In whatever form, the color or shade of a particular applied makeup formulation usually fades with time as the pigmented solid particles are rubbed off or abraded during daily activity. It has long been a desirable objective in the cosmetic arts to render the original application of makeup color "long wearing" or renewable on a sustained basis without reapplication. In order to meet this objective of providing a cosmetic formulation having color renewable characteristics thereby giving it the desired "long wearing" look or quality, the present invention in a first preferred form contemplates dispersing the color component or pigmented solid in a liquid carrier, microencapsulating the liquid carrier dispersion to form a stable, dry, free flowing powder and then combining the resulting microcapsules with other compatible ingredients of a particular cosmetic base or vehicle.

In forming the initial dispersion, a selected colorant in the form of pigmented solid particles is mixed with a suitable liquid carrier in an amount ranging up to about 70% by weight of the total dispersion, i.e. the remainder of the dispersion comprises not less than about 30% by weight of the liquid carrier. As will be made more apparent below, this ratio may be adjusted however, depending upon the type of pigment selected and the type of liquid carrier selected, it being critical only that the viscosity of the resulting dispersion be less than about 4000 cps when measured at room temperature (26°C).

An increase in the amount of pigment relative to the liquid carrier generally will result in an increase in the viscosity of the dispersion leading to microcapsules that are too hard and gritty, whereas a decrease in pigment relative to the liquid may result in microcapsules that are too easily rupturable, or to less than desirable intensity of the release pigment or color. When utilizing a low viscosity liquid carrier (e.g. mineral oil) a pigment/liquid carrier ratio in the range of about 1:2 to about 1:1 has been found to achieve a good balance between color intensity, lack of "grittyness" and capsule integrity, and therefore, is mostly preferred.

Suitable pigments may comprise any organic or inorganic pigment or colorant approved for use in cosmetics by the CTFA and the FDA such as the lakes, iron oxides, titanium dioxide, iron sulfides, or other conventional pigments used in cosmetic formulations. Conventional approved dyes may also be used provided they are hydrophobic (water insoluble). The lakes (organic) and the iron oxides are particularly preferred because of their excellent color intensity.

The liquid carrier component of the initial dispersion must be inert, hydrophobic (water insoluble) and non-volatile, have a low viscosity and be capable of forming a colloidal suspension when the pigment granules are mixed therein. Any of the conventional oleophilic or oil compounds used in cosmetic formulations such as mineral oil, vegetable oil, peanut oil, lanolin oil, squalene, castor oil, isopropyl myristate, isopropyl palmitate, silicone oil, and diisopropyl dimerate, either alone or blended together, are suitable. However, because the viscosity of the initial dispersion prior to being microencapsulated must be carefully controlled within the desired range mentioned above, it has been found that a light or low viscosity mineral oil provides the best results. In this regard, a light mineral oil sold by Witco, PetrolRa, Pa., under the designation "Carnation White Mineral Oil" is mostly preferred. Nonetheless, it will be appreciated that the other oil compounds mentioned above as well as those commonly used in the cosmetic arts may be employed instead so long as their viscosity is modified or controlled sufficiently to form a dispersion having a viscosity not more than about 4000 cps when measured at room temperature.

In accordance with the invention, the pigmented solid components and the liquid carrier are mixed together at room temperature in a conventional mixing kettle and then passed one or more times through a conventional grinding mill (e.g. a "stone" mill) until the resulting dispersion has a rich, creamy consistency with high gloss. Sufficient grinding of the dispersion will be achieved when a sample thereof measured on a standard Hegeman gauge reads no less than 6 1/2 indicating a maximum particle size of less than 20 microns.

Since grinding of the pigment/liquid carrier phase in the grinding mill will tend to increase somewhat the viscosity of the dispersion; and furthermore, since the particle size of different pigments vary, it will be necessary to adjust the pigment/oil ratio up or down depending upon the different pigments employed for a given liquid carrier. This is illustrated in Table 1 below which shows the relative proportions of pigment and liquid carrier by percent weight which must be employed to achieve ground dispersions within the desired viscosity range comprising a range of different preferred pigments and the preferred liquid carrier (Carnation White Mineral Oil), respectively.

TABLE I

| DISPERSION | | | |
|---|---|---|---|
| PIGMENT | %PIGMENT | %CARRIER* | VISCOSITY(cps)26°C |
| D&C Red 7 Calcium Lake | 40 | 60 | 1550 |
| D&C Red 30 Talc Lake | 33 | 67 | 3500 |
| D&C Red 6 Barium Lake | 45 | 55 | 1800 |
| Russet Iron Oxide | 50 | 50 | 2200 |
| Yellow Iron Oxide | 40 | 60 | 2500 |
| Brown Iron Oxide | 50 | 50 | 2000 |

*Carnation White Mineral Oil

Whichever preferred pigment of Table 1 is utilized, it will be appreciated that the initial dispersion is characterized by a viscosity of less than about 4000 cps when measured at room temperature (26°C), substantially uniform particle size distribution less than about 20 microns, and a substantially complete wetting of each particle by the liquid carrier (i.e. no agglomeration).

In accordance with the invention, the ground pigment/liquid carrier dispersion is microencapsulated to form stable, dry, free flowing powder of micro-sized particles whose diameter ranges from about 2 to about 20 microns. It has been found that microcapsules having a diameter larger than 20 microns results in an unacceptable cosmetic product by feeling rough or gritty on the skin.

The preferred method of microencapsulation is by the technique of coacervation. Under this method, which is well known and understood, a liquid dispersion is emulsified in a continuous, external aqueous phase to form micro-sized droplets and a complex of colloidal material added to the external phase is reacted upon in such a way to form a deposit on and around each droplet thereby forming an outer wall or shell. In the present case, the preferred colloids forming the outer wall or shell of each microcapsule comprise gelatin and gum arabic, the internal phase or core of which comprises the pigment/liquid carrier dispersion. After formation of the outer shells, the temperature of the aqueous coacervating solution is lowered causing gelation and hardening of the shell wall material. Further hardening may be accomplished by treating with a condensate polymer (e.g. urea formaldehyde) and applying a cross-linking agent (e.g. glutaraldehyde) to some or all of the available amino sites on the surface of the gelatin/gum arabic wall. Polymerisation induced by the cross-linking agent causes the capsule's wall to harden sufficiently so that the microencapsules may be removed from the continuous external phase and dehydrated to form a stable, dry, free flowing powder capable of being handled easily and further processed to make cosmetic products without undue rupture of the microcapsules.

When the preferred coacervation method is used to form the microcapsules, the size of the cores, as well as the wall thickness and strength of the outer walls or shells, may be controlled precisely by altering such factors as the pH of the aqueous phase, the relative concentration of the colloids in the aqueous phase, the degree of agitation of the coacervation solution, the temperature and duration of the reaction, the degree of cross-linking, and so on, all as is fully known and understood in the art.

Accordingly, these details form no part of and are outside the scope of the present invention. Suffice it to say, in practicing the invention, the initial pigment dispersion is microencapsulated to form capsules ranging in size from about 2 micronsto about 20 microns, wherein the internal phase or core of pigment/oil preferably comprises from about 80% to about 90% of each capsule by weight; the outer shell components, i.e. gelatin and gum arabic, each comprises about 3% to about 5% by weight; the condensate polymer (urea formaldehyde) comprises about 4.00% to about 8.00% by weight; and the cross-linking agent (glutaraldehyde) comprises about 0.10% to about 0.50% by weight.

It has been found in accordance with the invention that microcapsules of the foregoing composition containing a core of pigment dispersed in a liquid carrier wherein the resulting dispersion has a viscosity of less than about 4000 cps when measured at room temperature are easily rupturable when subjected to shear forces (e.g. rubbing on the skin, eating, stroking of the cheeks, etc.), yet are capable of maintaining their integrity and withstanding rupture during the handling and processing steps necessary to form the novel cosmetic formulations contemplated herein.

Following microencapsulation, the encased pigment/oil dispersion, now in the form of an easily handled, stable, dry free flowing powder is added to a compatible, anhydrous vehicle or base to form cosmetic products having a "long wearing" or renewable color characteristic. Owing to the relatively high concentration of pigment in each microcapsule core, a surprisingly small amount of microencapsulated pigment is necessary in each cosmetic vehicle to provide high intensity color renewability when the microcapsules are "activated", i.e. ruptured by being subjected to shear forces.

In making a preferred form of "long wearing" lipstick, for example, about 5% of the lipstick base by weight may comprise microencapsulated pigment according to the invention. In a preferred formula for a "long wearing" powder blush, the microencapsulated pigment content may be about 10% of the vehicle by weight. Nonetheless, it will be understood that as much microencapsulated pigment may be added to a selected cosmetic vehicle or base as desired so long as the cosmetic form in question is still capable of being applied to the skin and the microcapsules do not feel granular or are otherwise apparent. In general, the particular preferred amount of microencapsulated pigment employed may range up to about 60% of the base vehicle with an amount in the range of about 2% to about 20% being mostly preferred. In use, light rubbing of the cosmetic preparation after application, or movement of the skin resulting from normal daily activities (e.g., smiling, pursing of the lips, eating, kissing, stroking of the cheeks) activates new color by causing rupture of the microcapsules and a release of pigment into the cosmetic base sufficient to produce a perceptible "long wearing" affect.

PREFERRED EMBODIMENTS OF THE INVENTION

Other means suitable for making the pigment dormant and activatable by shear forces may be employed instead. Thus, in a preferred embodiment of the invention, it has been found that the pigment to be released on a sustained basis may be rendered activatable by entrapment on or within a solid substrate combined with the cosmetic vehicle or base in question. The preferred entrapping medium comprises a fine particle-sized, solid particulate that is inert, hydrophobic, and insoluble and which contains many pores or sites giving it a high surface area per unit volume. In general, any particulate approved for use in cosmetics and meeting the foregoing criteria is suitable.

Examples include polymethacrylate copolymers, silica beads, and porous nylon powders with the polymethacrylate copolymer sold by Dow Corning under the designation POLYTRAP Q5-6603 being mostly preferred. The mostly preferred entrapping medium consists of micro-sized particles of copolymer pressed together to form agglomerates (20-80 microns) which, in turn, are loosely clustered into aggregates of 200 to 1200 microns in size. The copolymer is available in the form of a stable, dry free flowing powder facilitating its use in cosmetic formulations.

Any of the pigments or dyes mentioned above may be loaded onto the polymethasrylate copolymer agglomerates by mixing the colorants with the copolymer powder in a suitable blender such as a Baker-Perkins disperser/granulator. In general, 1 to 2 parts pigment per part of copolymer are mixed together. It is believed that the pigment mechanically attaches to the irregular copolymer surface and lodges in the nooks and crannies thereof rather than be absorbed as in the case of liquid systems being loaded onto the copolymer. In order to maintain the pigment or colorant stable on the copolymer, in accordance with the invention, a thin film or layer is applied to the pigment loaded copolymer substrate. Generally, any non-volatile, oleophilic liquid approved for use in cosmetics is suitable for this purpose such as the oil compounds mentioned above in connection with the light (low viscosity) mineral oil such as the "Carnation White" form available from Witco being mostly preferred. The amount of liquid adherent is not critical so long as a sufficient amount is present to completely wet the pigment particles on the copolymer surface with due allowance being given for the high absorption capability of the particulate. Generally from .2 to 1.4 parts of liquid adherent to 1 part pigment is suitable. Based upon the copolymer content, the liquid adherent should range from about .25 to 1.0 parts for each part of the copolymer. The liquid adherent may be added to the pigment/copolymer blend in a standard mixer and blended thoroughly to disperse the liquid throughout the solids. If desired, suitable processing aids such as fumed silica may be added to the pigment before the liquid is added. After mixing these ingredients together, the pigment entrapped copolymer will be in the form of a stable, dry, free flowing powder.

The pigment entrapping powder may then be added to a conventional, compatible, anhydrous cosmetic base or vehicle to produce cosmetic formulations containing "activatable" dormant pigment suitable when activated or released to give the formulation a "long wearing" affect regarding its color or shade. In use, a slight rubbing of the cosmetic formula into the skin will tend to rupture or otherwise mechanically distort the aggregated polymethacrylate copolymer material thereby releasing and spreading the pigment/liquid adherent dispersion on the surface of the skin, thus activating the colorant and giving the formulation its "long wearing" or renewed color characteristic. The same results may be achieved by applying other shear forces, say, in the case of lipstick, for example, those occasioned by eating, pursing of the lips, kissing, smiling, and so on.

The foregoing preferred form of the invention will now be further illustrated by the following Example wherein all stated quantities are by percent weight.

## EXAMPLE

The encapsulated pigment was combined with a pigment entrapped substrate to form a powder "blush" having the following formulation:

|  |  |  |
|---|---|---|
|  | 30.00000% | Mica |
|  | 22.00000% | Talc |
|  | 9.00000% | Neopentaglycol Dicapryl Disaprate |
|  | 9.00000% | Bismuth Oxychloride |
|  | 4.00000% | Calcium Stearate |
|  | I.SOOOO% | Calcium Silicate |
|  | 1.50000% | Synthetic Wax (and) Corn Gluten Protein |
|  | 0.60000% | Wool Wax Alcohols |
|  | 0.25000% | D&C Red 7 Calcium Lake |
|  | 3.75000% | D&C Red 6 Barium Lake |
|  | O.I9000% | Iron Oxide Red |
|  | 0.04000% | Iron Oxide Yellow |
|  | 0.13000% | D&C Red 6 Barfum Lake |
|  | 10.04000% | Timiron MP-1001 |
|  | 3.00000% | Microencapsulated Pigment |
|  | 5.OOOOO% | Entrapped Pigment |
| Total: | IOO.OOOOO% |  |

The powder "blush" was applied from the pan via a brush applicator to the cheeks of a subject. As a result of normal daily activity e.g. smiling, eating, talking and stroking the cheeks, a quantity of the microcapsules and/or entrapped pigments were ruptured thereby releasing additional color to the cheeks. The additional color provided more-intense color and a perceived long lasting affect compared to the blush made in accordance with the same formulation, but not containing any microencapsulated and/or entrapped pigment. Moreover, the resulting palette of renewable color comprised two components, i.e. an intense component and a more subtly shaded component.

It is evident from the foregoing that the present invention in its broadest sense contemplates the provision of an activatable dormant pigment in a cosmetic formulation wherein activation of the dormant pigment by applied shear forces releases color into the formulation thereby renewing the original color of the formulation with the same or

a different shade and giving the formulation a "long wearing" affect with regard to color. By encapsulating the pigment or entrapping it on a substrate the pigment is rendered inactive or dormant until the application of shear forces. Thus, the term "activatable" as used in the specification and claims means the release of the pigment from its dormant state in the cosmetic formulation to an active state where it renews, reinforces, or enhances the intensity of the original color, or it juxtaposes a new or different color relative
to the original color thereby producing striking and aesthetic effects.

**Claims**

1. Color sustainable cosmetic formulation comprising a base phase having a characteristic color and microcapsules containing color pigments or dyes which microcapsules are dispersed in said base phase and which pigments are activatable when said microcapsules are subjected to physical forces so as to sustain said characteristic color of said base phase,
   **characterized in that**
   a medium is additionally dispersed in said base phase, said medium being capable of entrapping and/or releasing pigments or dyes by means of a solid substrate, preferably solid particulates.

2. Cosmetic formulation as claimed in claim 1,
   **characterized in that**
   said particulates render said color pigments of said microcapsules activatable after said microcapsules are subjected to said physical force.

3. Cosmetic formulation as claimed in claim 1 or 2,
   **characterized in that**
   said particulates are inert, hydrophobic and insoluble and comprise many pores or sites giving it a high surface area per unit volume.

4. Cosmetic formulation as claimed in one of the claims 1-3,
   **characterized in that**
   said particulates comprise microsized particles of polymethacrylate copolymer pressed together to form agglomerates of between 20 and 80 μm which in turn are loosely clustered into aggregates of between 200 and 1200 μm size.

5. Cosmetic formulation as claimed in any of the preceding claims,
   **characterized in that**
   said color pigment or dye is deposited on said particulates in an amount of about 0,2 part to about 2 part by weight of said pigment or dye for each part by weight of said particulate.

6. Cosmetic formulation as claimed in claim 4,
   **characterized in that**
   said particulates and pigments are coated by a film of a non-volatile oleophilic liquid.

7. Cosmetic formulation as claimed in claim 5,
   **characterized in that**
   a liquid of low viscosity is used which liquid is capable of forming a colloidal suspension when pigmented granules are mixed therein.

8. Cosmetic formulation as claimed in claims 5 or 6,
   **characterized in that**
   said liquid is coated in an amount of 0,6 to 0,9 parts by weight for each part of weight of said particulate.

9. Cosmetic formulation as claimed in one of the preceding claims,
   **characterized in that**
   said microcapsules also comprise a dispersion of said pigment or dye in a hydrophobic non-volatile, low viscosity liquid.

10. Cosmetic formulation as claimed in one of the claims 5-8,
    **characterized in that**
    said liquid is selected from the group consisting of mineral oil, vegetable oil, squalene, castor oil, isopropyl myristate, isopropyl palmitate, peanut oil, lanolin oil, silicone oil, diisopropyl dimerate, and combinations thereof.

11. Cosmetic formulation as claimed in claim 8 or 9,
**characterized in that**
said dispersion has a viscosity of less than 4000 cps at room temperature.

12. Cosmetic formulation as claimed in one of the preceding claims,
**characterized in that**
the size of said microcapsules is in the range of 2 to 20 m.

13. Cosmetic formulation as claimed in one of the preceding claims,
**characterized in that**
said pigment is dispersed in said base phase in the range of 2 to 20% by weight.

14. Use of the cosmetic formulation as claimed in one of the preceding claims for lipsticks.

15. Use of the cosmetic formulation as claimed in one of the preceding claims for blushes.

16. Use of the cosmetic formulation as claimed in one of the preceding claims for foundations.

17. Use of the cosmetic formulation as claimed in one of the preceding claims for face powder.

18. Use of the cosmetic formulation as claimed in one of the preceding claims for eyeshadow.

## Patentansprüche

1. Kosmetische Depotfarbformulierung, die eine Basisphase mit einer charakteristischen Farbe und Mikrokapseln aufweist, die Farbpigmente oder Farbstoffe enthalten, in der Basisphase dispergiert sind und aktivierbar sind, wenn die Mikrokapseln physischen Kräften unterworfen werden, um die charakteristische Farbe der Basisphase zu halten,
**dadurch gekennzeichnet, daß**
zusätzlich ein Medium in der Basisphase dispergiert wird, wobei das Medium in der Lage ist, Pigmente oder Farbstoffe mittels eines festen Substrats, bevorzugt fester Teilchensubstanzen einzuschließen und/oder freizusetzen.

2. Kosmetische Formulierung nach Anspruch 1,
**dadurch gekennzeichnet, daß**
die Teilchensubstanzen die Farbpigmente der Mikrokapseln aktivierbar machen, nachdem die Mikrokapseln der physischen Kraft unterworfen sind.

3. Kosmetische Formulierung nach Anspruch 1 oder 2,
**dadurch gekennzeichnet, daß**
die Teilchensubstanzen inert, hydrophob und unlöslich sind und viele Poren oder Stellen aufweisen, womit sie einen hohen Flächeninhalt pro Volumeneinheit erhalten.

4. Kosmetische Formulierung nach einem der Ansprüche 1-3,
**dadurch gekennzeichnet, daß**
die Teilchensubstanzen Teilchen aus einem Polymethacrylatcopolymeren in Mikrogröße aufweisen, die zusammengepreßt sind, um Agglomerate zwischen 20 und 80 $\mu$m zu bilden, die ihrerseits locker zu Aggregaten mit einer Größe zwischen 200 und 1200 $\mu$m aufweisen.

5. Kosmetische Formulierung nach einem der vorhergehenden Ansprüche,
**dadurch gekennzeichnet, daß**
das Farbpigment oder der Farbstoff an den Teilchensubstanzen in einer Menge von etwa 0,2 bis etwa 2 Gewichtsteilen des Pigments oder Farbstoffs für jedes Gewichtsteil der Teilchensubstanz aufgebracht wird.

6. Kosmetische Formulierung nach Anspruch 4,
**dadurch gekennzeichnet, daß**
die Teilchensubstanzen und Pigmente mit einem Film aus einer nichtflüchtigen, oleophilen Flüssigkeit beschichtet sind.

7. Kosmetische Formulierung nach Anspruch 5,
   **dadurch gekennzeichnet, daß**
   eine Flüssigkeit mit niedriger Viskosität verwendet wird, die zur Bildung einer Kolloidsuspension in der Lage ist, wenn Pigmentkörnchen damit vermischt werden.

8. Kosmetische Formulierung nach den Ansprüchen 5 oder 6,
   **dadurch gekennzeichnet, daß**
   die Flüssigkeit in einer Menge von 0,6 bis 0,9 Gewichtsteilen für jedes Gewichtsteil der Teilchensubstanz beschichtet ist.

9. Kosmetische Formulierung nach einem der vorhergehenden Ansprüche,
   **dadurch gekennzeichnet, daß**
   die Mikrokapseln auch eine Dispersion des Pigments oder Farbstoffs in einer hydrophoben, nichtflüchtigen Flüssigkeit mit niedriger Viskosität aufweisen.

10. Kosmetische Formulierung nach einem der Ansprüche 5-8,
    **dadurch gekennzeichnet, daß**
    daß die Flüssigkeit aus der Gruppe von medizinischem Öl, Pflanzenöl, Squalen, Castoröl, Isopropylmyristat, Isopropylpalmitat, Erdnußöl, Lanolinöl, Siliconöl, Diispropyldimerat und Kombinationen davon ausgewählt ist.

11. Kosmetische Formulierung nach einem der Ansprüche 8 oder 9,
    **dadurch gekennzeichnet, daß**
    die Dispersion eine Viskosität von weniger als 4000 cps bei Zimmertemperatur aufweist.

12. Kosmetische Formulierung nach einem der vorhergehenden Ansprüche,
    **dadurch gekennzeichnet, daß**
    die Größe der Mikrokapseln im Bereich von 2 bis 20 $\mu$m liegt.

13. Kosmetische Formulierung nach einem der vorhergehenden Ansprüche,
    **dadurch gekennzeichnet, daß**
    das Pigment in der Basisphase im Bereich von 2 bis 20 Gew.-% dispergiert ist.

14. Verwendung der kosmetischen Formulierung nach einem der vorhergehenden Ansprüche für Lippenstifte.

15. Verwendung der kosmetischen Formulierung nach einem der vorhergehenden Ansprüche für Rouge.

16. Verwendung der kosmetischen Formulierung nach einem der vorhergehenden Ansprüche für Grundierungscremes.

17. Verwendung der kosmetischen Formulierung nach einem der vorhergehenden Ansprüche für Gesichtspuder.

18. Verwendung der kosmetischen Formulierung nach einem der vorhergehenden Ansprüche für Lidschatten.

**Revendications**

1. Formulation cosmétique à couleur prolongée, comprenant une phase de base ayant une couleur caractéristique et des microcapsules contenant des pigments colorés ou des pigments, les microcapsules étant dispersées dans ladite phase de base, et les pigments étant activables quand lesdites microcapsules sont soumises à des forces physiques de façon à prolonger ladite couleur caractéristique de ladite phase de base, caractérisée en ce qu'un milieu est en outre dispersé dans ladite phase de base, ledit milieu étant capable de piéger et/ou de libérer des pigments ou des colorants au moyen d'un substrat solide, de préférence des particules solides.

2. Formulation cosmétique selon la revendication 1, caractérisée en ce que lesdites particules rendent lesdits pigments colorés desdites microcapsules activables après que lesdites microcapsules ont été soumises à ladite force physique.

3. Formulation cosmétique selon la revendication 1 ou 2, caractérisée en ce que lesdites particules sont inertes, hydrophobes et insolubles, et comprennent de nombreux pores ou sites qui leur confèrent une grande aire par volume unitaire.

**4.** Formulation cosmétique selon l'une des revendications 1 à 3, caractérisée en ce que lesdites particules comprennent des particules de très petite granulométrie, constituées d'un copolymère de polyméthacrylate, comprimées pour former des agglomérats de 20 à 80 $\mu$m, qui pour leur part sont réunis d'une manière lâche en agrégats de 200 à 1200 $\mu$m.

**5.** Formulation cosmétique selon l'une quelconque des revendications précédentes, caractérisée en ce que ledit pigment coloré ou colorant est déposé sur lesdites particules en une quantité d'environ 0,2 à environ 2 parties en poids dudit pigment ou colorant pour chaque partie en poids desdites particules.

**6.** Formulation cosmétique selon la revendication 4, caractérisée en ce que lesdites particules et lesdits pigments sont revêtus d'un film d'un liquide oléophile non volatil.

**7.** Formulation cosmétique selon la revendication 5, caractérisée en ce qu'on utilise un liquide ayant une faible viscosité, lequel liquide est à même de former une suspension colloïdale quand on y mélange des granulés pigmentés.

**8.** Formulation cosmétique selon les revendications 5 ou 6, caractérisée en ce que ledit liquide est appliqué en une quantité de 0,6 à 0,9 partie en poids par partie en poids desdites particules.

**9.** Formulation cosmétique selon l'une des revendications précédentes, caractérisée en ce que lesdites microcapsules comprennent aussi une dispersion dudit pigment ou colorant dans un liquide non volatil hydrophobe à faible viscosité.

**10.** Formulation cosmétique selon l'une des revendications 5 à 8, caractérisée en ce que ledit liquide est choisi dans l'ensemble comprenant les huiles minérales, les huiles végétales, le squalène, l'huile de ricin, le myristate d'isopropyle, le palmitate d'isopropyle, l'huile d'arachide, l'huile de lanoline, l'huile de silicone, l'ester diisopropylique de l'acide dimère et leurs combinaisons.

**11.** Formulation cosmétique selon la revendication 8 ou 9, caractérisée en ce que ladite dispersion a une viscosité inférieure à 4000 cP à la température ambiante.

**12.** Formulation cosmétique selon l'une des revendications précédentes, caractérisée en ce que la taille desdites microcapsules est comprise entre 2 et 20 $\mu$m.

**13.** Formulation cosmétique selon l'une des revendications précédentes, caractérisée en ce que ledit pigment est dispersé dans ladite phase de base en une quantité de 2 à 20 % en poids.

**14.** Utilisation de la formulation cosmétique selon l'une des revendications précédentes, pour des rouges à lèvres.

**15.** Utilisation de la formulation cosmétique selon l'une des revendications précédentes, pour un fard à joues.

**16.** Utilisation de la formulation cosmétique selon l'une des revendications précédentes, pour des fonds de teint.

**17.** Utilisation de la formulation cosmétique selon l'une des revendications précédentes, pour une poudre pour le visage.

**18.** Utilisation de la formulation cosmétique selon l'une des revendications précédentes, en tant qu'ombre à paupières.